# EUROPEAN PATENT APPLICATION

(11) **EP 3 808 350 A1**
(43) Date of publication of application: **21.04.2021**
(21) Application number: 19823161.5
(22) Date of filing: 18.06.2019
(51) Int. Cl.: A61K 31/5377, A61K 31/553, A61K 31/496, A61K 45/06, A61P 35/02, G01N 33/68, G01N 33/50

(54) **COMPOSITION COMPRISING FLT3 INHIBITOR AS EFFECTIVE INGREDIENT FOR INHIBITING DRUG RESISTANCE IN CHRONIC MYELOGENOUS LEUKEMIA**

(30) Priority: 18.06.2018 KR 20180069510
(71) Applicant: Industry-Academic Cooperation Foundation, Yonsei University, Seoul 03722 (KR)
(72) Inventor: PARK, Hyun Woo, Seoul 03730 (KR); SHIN, Jieun, Seoul 03309 (KR)
(74) Representative: V.O.
(86) International application number: PCT/KR2019/007350
(87) International publication number: WO 2019/245269

(57) **Abstract**

The present disclosure provides a composition for inhibiting tolerance to a drug for chronic myelogenous leukemia (CML), which contains an FLT3 (FMS-like tyrosine kinase 3) inhibitor as an active ingredient.

## Description

### [Technical Field]

The present disclosure relates to a composition for inhibiting tolerance to a drug for chronic myelogenous leukemia, which contains an FLT3 inhibitor as an active ingredient.

### [Background Art]

Leukemia is a cancerous disease of bone marrow and blood. Leukemia can be classified into four types: chronic myelogenous leukemia, acute myelogenous leukemia, chronic lymphocytic leukemia and acute lymphocytic leukemia.

The acute type of myelogenous leukemia that proceeds quickly is called acute myelogenous leukemia or AML. The chronic type of myelogenous leukemia which proceeds gradually or less invasively is called chronic myelogenous leukemia or CML.

Chronic myelogenous leukemia (CML) is a blood cancer occurring as the clones of hematopoietic stem cells having the Philadelphia chromosome proliferate abnormally.

Chronic myelogenous leukemia is caused by the Philadelphia chromosome resulting from the translocation of chromosome 9 and chromosome 22.

As a result of the chromosomal translocation, the ABL gene on chromosome 9 is fused with the BCR gene on chromosome 22, and the fused BCR-ABL gene generates a BCR-ABL fusion protein having an abnormal tyrosine kinase activity. The BCR-ABL tyrosine kinase induces abnormal cell division.

The Philadelphia chromosome forms and continuously activates the BCR-ABL fusion protein, which is the cause of CML. The abnormal BCR-ABL protein is expressed in 90% or more of patients with chronic myelogenous leukemia (CML), and the continuous activation of BCR-ABL further accelerates leukemia.

As a drug for treating chronic myelogenous leukemia, imatinib is sold under the brand name Gleevec. But, various mutant species having resistance to Gleevec are being reported recently.

T315I, known as a gatekeeper mutant species, is not treated with Gleevec, nor with nilotinib, dasatinib, bosutinib, etc., which are known as second-generation BCR-ABL inhibitors.

Meanwhile, it is known that the malignancy of chronic myelogenous leukemia is increased through three phases.

The three phases of chronic myelogenous leukemia are chronic phase, accelerated phase and blast crisis. In the blast crisis CML (bc-CML), drug tolerance is very high and survival rate is very low as the disease proceeds very rapidly.

Meanwhile, 90% or more of patients with acute myelogenous leukemia (AML) exhibits expression of FLT3 by undifferentiated cells, unlike CML.

About 30-40% of AML patients are known to have activating mutation of FLT3. The FLT3 mutation is the most common mutation in AML patients.

Since CML and AML have different mechanisms as described above, different drugs are prescribed for the diseases.

The present inventors have studied various methods for treating CML, which difficult to treat due to drug tolerance. In doing so, they have identified that the expression of FLT3 mRNA is increased rapidly as the malignancy of CML is increased. The present disclosure aims at providing a new target for treating patients with blast crisis CML, which exhibits very low survival rate due to drug tolerance.

### [Disclosure]

### [Technical Problem]

The present disclosure is directed to clearly elucidating the relationship between FLT3 and drug in treating chronic myelogenous leukemia (CML) and providing a fundamental method for treating CML, which is difficult to treat due to drug tolerance.

### [Technical Solution]

In an aspect, the present disclosure provides a composition for inhibiting tolerance to a composition for preventing or treating chronic myelogenous leukemia (CML), which contains an FLT3 (FMS-like tyrosine kinase 3) inhibitor as an active ingredient.

In an exemplary embodiment, the FLT3 inhibitor may be one or more inhibitor selected from the group consisting of ponatinib, quizartinib, midostaurin, dovitinib, amuvatinib, tandutinib, sorafenib, gilteritinib, crenolanib and pacritinib.

In an exemplary embodiment, the chronic myelogenous leukemia (CML) may be blast crisis chronic myelogenous leukemia (bc-CML).

In another aspect, the present disclosure provides a pharmaceutical composition for treating chronic myelogenous leukemia, which contains an FLT3 inhibitor as an active ingredient.

In an exemplary embodiment, the FLT3 inhibitor may be one or more inhibitor selected from the group consisting of ponatinib, quizartinib, midostaurin, dovitinib, amuvatinib, tandutinib, sorafenib, gilteritinib, crenolanib and pacritinib.

In an exemplary embodiment, the FLT3 inhibitor may be administered together with a tyrosine kinase inhibitor.

In an exemplary embodiment, the tyrosine kinase inhibitor may be one or more selected from the group consisting of imatinib, dasatinib, nilotinib, bosutinib and ponatinib.

In an exemplary embodiment, the chronic myelogenous leukemia (CML) may be drug-tolerant.

In another aspect, the present disclosure provides a method for screening a composition for inhibiting tolerance to a composition for preventing or treating chronic myelogenous leukemia (CML), which includes: (a) contacting a biological sample containing FLT3-expressing cells with a test agent; and (b) measuring expression level or activity of the FLT3 in the sample, wherein, if expression level or activity of the FLT3 is decreased, the test agent is determined as a composition for inhibiting tolerance to a composition for preventing or treating chronic myelogenous leukemia.

In another aspect, the present disclosure provides a composition for predicting a responsiveness of a composition for preventing or treating chronic myelogenous leukemia (CML), comprising an agent which measures the expression level of FLT3 protein or a gene encoding the same as an active ingredient.

In another aspect, the present disclosure provides a composition for diagnosing blast crisis chronic myelogenous leukemia (bc-CML), comprising an agent which measures the expression level of FLT3 protein or a gene encoding the same as an active ingredient.

### [Advantageous Effects]

The present disclosure provides a method for effectively inhibiting the drug tolerance of a CML patient based on clear understanding of the molecular mechanism of FLT3 and may be usefully applied for treatment, diagnosis, research, etc. of chronic myelogenous leukemia.

It should be understood that the effects of the present disclosure are not limited to those described above but include all the effects that can be inferred from the constitution of the present disclosure described in the detailed description or claims of the present disclosure.

### [Brief Description of Drawings]

FIG. 1 shows a result of measuring the change in expression of FLT3 and TAZ mRNAs depending on the progress of chronic myelogenous leukemia.
FIG. 2 shows a result of elucidating the relationship between FLT3 and drug tolerance in CML-mimicking cells.
FIG. 3 shows a result of measuring the expression level of the FLT3 and TAZ proteins in CML-mimicking cells depending on drug tolerance.
FIGS. 4 and 5 show a result of observing the apoptotic effect of treatment with an FLT3 inhibitor (quizartinib) and/or a therapeutic agent for CML in CML-mimicking cells having drug tolerance.
FIGS. 6 and 7 show a result of observing the apoptotic effect of treatment with an FLT3 inhibitor (midostaurin) and/or a therapeutic agent for CML in CML-mimicking cells having drug tolerance.
FIGS. 8a-8c show a result of testing the inhibition of drug tolerance and apoptotic activity of FLT3 inhibitors, ponatinib and quizartinib (FIG. 8a), midostaurin and sorafenib (FIG. 8b), and gilteritinib and crenolanib (FIG. 8c) for K562 cells.
FIGS. 9a-9e show a result of investigating the inhibition of drug tolerance and apoptotic activity of inhibitors against JAK, STAT3, TAZ, TEAD and CD36, which are sub-regulatory factors of FLT3. The result of conducting MTT assay after treating FLT3-transformed K562 cells with a JAK inhibitor (FIG. 9a), an STAT3 inhibitor (FIG. 9b), a TAZ inhibitor (FIG. 9c), a TEAD inhibitor (FIG. 9d) and a CD36 inhibitor (FIG. 9e) together with imatinib is shown.
FIGS. 10a-10e show a result of investigating the inhibition of drug tolerance and apoptotic activity of inhibitors against JAK, STAT3, TAZ, TEAD and CD36, which are sub-regulatory factors of FLT3. The result of conducting MTT assay after treating FLT3-transformed K562 cells with a JAK inhibitor (FIG. 10a), an STAT3 inhibitor (FIG. 10b), a TEAD inhibitor (FIG. 10c), a TAZ inhibitor (FIG. 10d) and a CD36 inhibitor (FIG. 10e) without imatinib is shown.

### [Best Mode]

Hereinafter, the present disclosure is described referring to the attached drawings. However, the present disclosure may be embodied in various different forms and is not limited to the exemplary embodiments described herein. When a portion is described to "include" a specific element, it does not mean that another element is excluded but means that there may be another element, unless specially stated otherwise.

Unless defined otherwise, the present disclosure may be performed by techniques common in the field of molecular biology, microbiology, protein purification, protein engineering, DNA sequencing and recombinant DNA technology within the level of those skilled in the art. The techniques are known to those skilled in the art are described in many standardized textbooks and references.

Unless defined otherwise, all the technical and scientific terms used in the present disclosure have the same meanings commonly understood by those of ordinary skill in the art.

A variety of scientific dictionaries including the terms used in the present disclosure are known and available in the art. Some methods and materials are described in the present disclosure although any methods and materials similar or equivalent to those described in the present disclosure can be used in the practice or testing of the present disclosure. The present disclosure is not limited to particular methodology protocols or reagents since they can vary depending on the context they are used by those skilled in the art. Hereinafter, the present disclosure is described in more detail.

In an aspect, the present disclosure provides a composition for inhibiting tolerance to a composition for preventing or treating chronic myelogenous leukemia (CML), which contains an FLT3 (FMS-like tyrosine kinase 3) inhibitor as an active ingredient.

The chronic myelogenous leukemia (CML) may be blast crisis chronic myelogenous leukemia (bc-CML).

The "blast crisis chronic myelogenous leukemia (bc-CML)" refers to a phase of chronic myelogenous leukemia. The chronic myelogenous leukemia may proceed through a chronic phase, an accelerated phase and a blast crisis. The blast crisis proceeds similarly to acute leukemia. The time period from the chronic phase to the blast crisis varies depending on individuals, from a few years to 10 years or longer. It is known that it takes 2.5-3 years to progress to the blast crisis in 75-80% of cases.

The blast crisis chronic myelogenous leukemia may be characterized by an increased population of granulocyte-macrophage progenitor (GMP)-like cells having acquired self-renewal capacity not shown in normal or chronic-phase (CP) GMPs.

The blast crisis chronic myelogenous leukemia (bc-CML) is difficult to treat with the existing therapeutic agent for CMLs because the drug tolerance is very high and the disease proceeds very rapidly. However, The present inventors have identified that the drug tolerance can be alleviated remarkably by treating with an FLT3 inhibitor.

The "FLT3 (FMS-like tyrosine kinase 3)" ligand is a cytokine which affects the development of the multiple hematopoietic system. The effect arises as FLT3L binds to the FLT3 receptor, and can be explained from the relationship between STK-1 and fetal liver kinase-2 (flk-2), which are receptor tyrosine kinases (RTKs) expressed in hematopoietic stem cells and progenitor cells.

The FLT3 gene encodes membrane-spanning class III RTK, which plays an important role in proliferation, differentiation and apoptosis of cells during normal hematopoiesis.

The FLT3 ligand is expressed in marrow stromal cells and other cells and synergizes with other growth factors to stimulate the proliferation of stem cells, progenitor cells, dendritic cells and natural killer cells.

Whereas FLT3 expression is restricted to early progenitor cells in normal bone marrow, FLT3 may be expressed at high levels or FLT3 mutations may cause an uncontrolled induction of the FLT3 receptor and downstream molecular pathways in blood cancer.

The acute myelogenous leukemia (AML) is the most prevalent form of adult leukemia and represents 15-20% of childhood leukemia.

Unlike CML, cancer is inducted by the mutations of the FLT3 protein in 30% of patients with acute myelogenous leukemia (AML), and 90% or more of AML patients show FLT3 expression in undifferentiated cells.

To date, there is no strong evidence that either the kinase domain point mutation or the overexpressed wild-type receptor is the cause of the disease. However, it is reported that FLT3 expression may contribute to the progression of the disease, and this building preclinical and clinical evidence has led to the development of a number of FLT3 inhibitors which are currently being evaluated in the preclinical and clinical setting.

Accordingly, drugs inhibiting the activity of FLT3 have been developed and are being tested in clinical trials for treatment of AML. However, they have not been used for treatment of CML, and nothing is known about the relationship between FLT3 and drug tolerance.

As a result of analyzing mRNA expression levels of patients with chronic myelogenous leukemia depending on phases, The present inventors have identified that FLT3, which has been reported to be expressed only in AML, increases rapidly with the progression of CML and aim at utilizing FLT3 as a new anticancer drug target for CML.

The "drug tolerance" refers to tolerance to a specific drug or a phenomenon whereby the anticancer effect of a specific drug is not achieved due to tolerance to the drug after long-term medication.

The "chronic myelogenous leukemia (CML)" refers to a hematological stem cell disease caused by increased and unregulated growth of myeloid cells in the bone marrow and excessive accumulation of white blood cells, and includes a disease caused by abnormal proliferation of hematopoietic stem cells having the Philadelphia chromosome in the bone marrow.

The "inhibitor" may refer to substance which inhibits or delays a biological pathway in which the FLT3 protein is involved by inhibiting the expression or lowering the activity of FLT3.

The FLT3 inhibitor is sufficient as long as it can inhibit the activity of FLT3 by interacting with it. The inhibitor may be one or more selected from the group consisting of an antibody or an antigen-binding fragment thereof, an aptamer, a siRNA, a shRNA, a microRNA, an inhibitory compound and a pharmaceutically acceptable salt thereof, although not being limited thereto.

The "antibody" includes a monoclonal antibody, a chimeric antibody thereof, a humanized antibody and a human antibody. In addition, antibodies previously known in the art may be included in addition to novel antibodies. The antibody may be a full-length antibody having two heavy chains and two light chains or a functional fragment of the antibody molecule, as long as it has the property of specifically recognizing the FLT3 protein. The functional fragment of the antibody molecule refers to a fragment at least having an antigen-binding function, and may be Fab, F(ab'), F(ab')2 or Fv.

The "siRNA" refers to a short double-stranded RNA capable of inducing the RNAi (RNA interference) phenomenon by cleaving a specific mRNA. It may consist of a sense RNA strain having a sequence homologous to the mRNA of a target gene and an antisense RNA strain having a sequence complementary thereto. Because the siRNA can inhibit the expression of the target gene, it can be provided as an effective tool for gene knockdown or gene therapy.

The siRNA is not limited to one in which double-stranded RNA portions are completely paired, and may include a non-paired portion due to a mismatch (the corresponding base is not complementary), a bulge (there is no corresponding base on one chain), etc. The siRNA may have either a blunt end or a cohesive end as long as it can effectively inhibit the expression of the target gene through RNAi.

As the cohesive end, both a 3'-end protruding structure and a 5'-end protruding structure are possible. The number of protruding bases is not limited. In addition, the siRNA may include a low-molecular-weight RNA (e.g., a RNA molecule such as tRNA, rRNA or viral RNA or an artificial RNA molecule) at a protruding portion at one end in a range that can main the effect of inhibiting the expression of the target gene.

The siRNA does not have to have cleavage structures on both ends, and may have a stem-loop structure in which one terminal region of a double-strained RNA is connected by a linker RNA.

The siRNA may be a complete form having a polynucleotide pairing i.e., a form in which siRNA synthesized directly in vitro is introduced into cells, a form from which a single-chain polynucleotide can be induced from a single-chain oligonucleotide fragment and its reverse complement are separated by a spacer after being administered in vivo, or a form in which a siRNA expression vector or a PCR-induced siRNA expression cassette is transformed or transfected into cells so that the siRNA is expressed in the cells.

The "shRNA" is intended to overcome the disadvantages of the siRNA, i.e., high cost of biosynthesis, short-term maintenance of RNA interference effect due to low cell transfection efficiency, etc. It may be expressed after being introduced into cells from a promoter of RNA polymerase III using an adenovirus, lentivirus or plasmid expression vector system. The shRNA may induce silencing of a target gene after being converted to a siRNA with an accurate structure by a siRNA-processing enzyme (Dicer or RNase III) existing in cells.

In an exemplary embodiment, the FLT3 inhibitor may be a small-molecule compound, e.g. one or more inhibitor selected from the group consisting of quizartinib, midostaurin, dovitinib, amuvatinib, tandutinib, gilteritinib and pacritinib, although not being limited thereto.

The "quizartinib (AC220)" is an inhibitor that inhibits FLT3. It is a leading candidate substance for acute myelogenous leukemia, which was designed as a liquid oral form and developed by Ambit Biosciences (USA).

In another aspect, the present disclosure also provides a pharmaceutical composition for preventing or treating chronic myelogenous leukemia, which contains an FLT3 inhibitor as an active ingredient.

In the present disclosure, the term "treatment" means (a) inhibition of the development of a disorder, a disease or a symptom; (b) alleviation of a disorder, a disease or a symptom; or (c) removal of a disorder, a disease or a symptom. The FTL3 inhibitor of the present disclosure not only improves drug tolerance by inhibiting FTL3 which is highly expressed in chronic myelogenous leukemia, specifically in blast crisis chronic myelogenous leukemia, but also inhibits, removes or alleviates the symptoms induced by excessive proliferation of myeloid cells and white blood cells by delaying the rapid progress of the disease. Accordingly, the composition of the present disclosure may be used as a therapeutic composition for chronic myelogenous leukemia by itself or may be administered together with other pharmacological ingredient as a therapeutic adjuvant for chronic myelogenous leukemia. Therefore, in the present disclosure, the term "treatment" or "therapeutic agent" includes the meanings of "therapeutic support" or "therapeutic adjuvant".

In the present disclosure, the term "prevention" means inhibition of the onset of a disorder or a disease in a subject who is not diagnosed with the disorder or disease but has a risk of having the disorder or disease.

In the present disclosure, the term "administration" or "administer" means directly administering a therapeutically effective amount of the composition of the present disclosure to a subject, so that the same amount is formed in the body of the subject. The "therapeutically effective amount" of the composition means an amount of the composition sufficient to provide a therapeutic or prophylactic effect to a subject to which the composition is to be administered and, therefore, includes "prophylactically effective amount". In the present disclosure, the term "subject" includes human, mouse, rat, guinea pig, dog, cat, horse, cow, pig, monkey, chimpanzee, baboon or rhesus monkey, without limitation. Specifically, the subject of the present disclosure is human.

In a specific exemplary embodiment of the present disclosure, the composition of the present disclosure is administered together with a tyrosine kinase inhibitor. For the co-administration, the FLT3 inhibitor and the tyrosine kinase inhibitor may be administered simultaneously or sequentially with appropriate order and time interval as either a combined composition or individual pharmaceutical compositions.

The "tyrosine kinase" is a protein capable of transferring a phosphate group from ATP to the tyrosine residue of a protein, and may play an important role in communicating signals and regulating cellular activity, e.g. cell division.

The tyrosine kinase may be BCR-ABL tyrosine kinase. The BCR-ABL tyrosine kinase may be produced from a BCR-ABL fused gene wherein the ABL gene on chromosome 9 is fused with the BCR gene on chromosome 22 due to the translocation of human chromosome 9 and chromosome 22 (t(9;22)(q34;q11)).

The "tyrosine kinase inhibitor" may be a drug having the activity of inhibiting a tyrosine kinase. For example. It may be BCR-ABL tyrosine kinase inhibitor.

Specifically, the tyrosine kinase inhibitor may be one or more inhibitor selected from the group consisting of imatinib, dasatinib, nilotinib, bosutinib and ponatinib. However, it is not specially limited as long as it can be used for treating chronic myelogenous leukemia by inhibiting a tyrosine kinase.

The pharmaceutical composition may be administered orally or parenterally. The pharmaceutical composition may be administered via any general routes as long as the target tissue can be reached. For example, the pharmaceutical composition may be administered orally, intraperitoneally, intravenously, intramuscularly, subcutaneously, intradermally, intranasally, intrapulmonarily, intrarectally, intracavitarily, intraabdominally or intradurally, although not being limited thereto.

The FLT3 activity inhibitor may be formulated together with a suitable amount of a pharmaceutically acceptable vehicle or carrier in order to provide an adequate administration form. And, the pharmaceutical composition may further contain a carrier, an excipient or a diluent used to prepare pharmaceutical compositions.

The carrier, excipient or diluent may be lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate or mineral oil, although not being limited thereto.

In addition, the composition may be prepared into a formulation for oral administration such as a powder, a granule, a tablet, a capsule, a suspension, an emulsion, a syrup, an aerosol, etc., a formulation for external use, a suppository or a sterilized injectable solution.

Solid formulations for oral administration include a tablet, a pill, a powder, a granule, a capsule, etc. These solid formulations may be prepared by mixing α-linolenic acid derived from perilla oil and fractions thereof with at least one excipient, e.g., starch, calcium carbonate, sucrose, lactose, gelatin, etc. In addition to the excipient, a lubricant such as magnesium stearate or talc may be used.

Liquid formulations for oral administration include a suspension, a solution for internal use, an emulsion, a syrup, etc. In addition to a simple diluent such as water and liquid paraffin, various excipients, e.g., a humectant, a sweetener, an aromatic, a preservative, etc. may be included.

As formulations for parenteral administration, a sterilized aqueous solution, a non-aqueous solution, a suspension, an emulsion, a lyophilized formulation or a suppository may be used. As the non-aqueous solution or suspension, propylene glycol, polyethylene glycol, a vegetable oil such as olive oil, or an injectable ester such as ethyl oleate may be used. As a base of the suppository, Witepsol, macrogol, Tween 61, cocoa butter, laurin butter or glycerogelatin may be used.

The pharmaceutical composition containing the FLT3 activity inhibitor as an active ingredient may be administered to a subject with a pharmaceutically effective amount.

The "pharmaceutically effective amount" refers to an amount sufficient to treat a disease at a reasonable benefit/risk ratio applicable to medical treatment. An effective dose level may be determined depending on the type and severity of a disease of a patient, drug reactivity, drug sensitivity, administration time, administration route, excretion rate, treatment period, co-administered drugs and other factors well known in the medical field.

The pharmaceutical composition may be administered as an individual therapeutic agent or in combination with other therapeutic agents. The co-administration with an existing therapeutic agent may be carried out sequentially or simultaneously, and may be carried out with single or multiple dosages. Specifically, the pharmaceutical composition may be administered with a minimum possible amount capable of obtaining the greatest effect without side effects in consideration of all the factors described above, and the amount may be easily determined by those skilled in the art.

In another aspect, the present disclosure also provides a method for screening a composition for inhibiting tolerance to a composition for preventing or treating chronic myelogenous leukemia (CML), which includes: (a) contacting a biological sample containing FLT3-expressing cells with a test agent; and (b) measuring the expression level or activity of the FLT3 in the sample, wherein, if expression level or activity of the FLT3 is decreased, the test agent is determined as a composition for inhibiting tolerance to a composition for preventing or treating chronic myelogenous leukemia.

The "contact" is a common meaning and may mean combining two or more agents (e.g., two polypeptides) or binding the agent (e.g., a protein) to cells.

For example, two or more agents may be combined or a test agent may be bound to cells or a cell lysate in a test tube or another container. Also, two polypeptides may be contacted with cells or a cell lysate by co-expressing a recombinant polypeptide encoding two polypeptides in the cells.

The "biological sample" may be any sample obtained from a mammal including human, which includes FLT3-expressing cells, and may include a tissue, an organ, a cell or a cell culture, although not being limited thereto.

The "agent" or "test agent" may include any substance, molecule, element, compound, entity or a combination thereof. Examples may include a protein, a polypeptide, a small organic molecule, a polysaccharide, a polynucleotide, etc. The agent may be a natural product, a synthetic compound, a chemical compound, or a combination of two or more of them. Unless specified otherwise, the terms agent, material and compound may be used interchangeably.

The screening method may use various biochemical and molecular biological technologies known in the art [Sambrook et al., Molecular Cloning: A Laboratory Manual, Cold Spring Harbor Press, N.Y., Second (1998) and Third (2000) Editions; Ausubel et al., Current Protocols in Molecular Biology, John Wiley & Sons, Inc., New York (1987-1999)]. For example, the screening method may be carried out using various methods known in the art, including in-vitro protein-protein binding assay (in-vitro pull down assay), EMSA, immunoassay for protein binding, functional assay (phosphorylation assay, etc.), yeast two-hybrid assay, non-immunoprecipitation assay, immunoprecipitation western blot assay, immuno-co-localization assay, etc.

The compound used in the screening may be a low-molecular-weight compound having a therapeutic effect. For example, the low-molecular-weight compound may have a molecular weight of about e.g., 400 Da, 600 Da, 800 Da or 1000 Da. The compound may constitute a portion of a compound library depending on purposes, and the number of compounds constituting the library may vary from dozens to millions.

The compound library may include peptides, peptoids and other cyclic or linear oligomeric compounds, and low-molecular-weight compounds based on a template, such as benzodiazepines, hydantoins, biaryls, carbocyclic and polycyclic compounds (e.g., naphthalene, phenothiazine, acridine, steroid, etc.), carbohydrates and amino acid derivatives, dihydropyridines, benzhydryls and heterocyclic compounds (e.g., triazine, indole, thiazolidine, etc.), although not being limited thereto.

In addition, a biologic may be used in the screening method. The biologic refers to a cell or a biomolecule, and means a protein, a nucleic acid, a carbohydrate, a lipid or a substance produced using a cellular system in vivo or in vitro.

The biomolecule may be used either alone or in combination with another biomolecule or a cell. For example, the biomolecule may include a polynucleotide, a peptide, an antibody, or a protein or biological organic material found in blood plasma.

The type and concentration of the cells and the amount, type, etc. of the materials used in the screening method may vary depending on the specific experimental procedure and the type of the test agent, and may be selected adequately by those skilled in the art. As a result of the experiment, a test agent which decreases the expression level or activity of FLT3 as compared to a control group not contacted with the test agent may be screened as a candidate substance.

In the present disclosure, the term "decrease in expression level or activity" means that the expression level of FLT3 or the intrinsic function of FLT3 in vivo is decreased to such an extent that the therapeutic efficiency of CML is improved to a measurable level as the drug tolerance induced by FLT3 is significantly inhibited. Specifically, the activity or expression level may be decreased by about 99% or less, about 95% or less, about 90%, about 85%, about 80%, about 75%, about 70%, about 65% or less, about 60% or less, about 55%, about 50% or less or 45% or less, as compared to a control group, although not being limited thereto.

The test agent may be assayed first in order to investigate whether it has an ability of regulating the biological activity of FLT3. Specifically, in the first assay, a modulating agent regulating the biological activity of the polypeptide may be elucidated by analyzing the biological activity of FLT3 in the presence of the test agent.

In the first assay, the regulation of various biological activities of FLT3 may be assayed. For example, it may be assayed whether the test agent has the activity of regulating the expression level of FLT3, e.g., transcription or translation. In addition, it may be assayed whether the test agent has the activity of regulating the intracellular level or stability of FLT, e.g. post-translational modification or hydrolysis.

After the modulating agent associated with the biological activity of FLT3 has been elucidated through the first assay, it may be assayed secondly whether the test agent can improve the drug tolerance of chronic myelogenous leukemia.

In the first and second assays, intact FLT3 or a fragment, analogue or functional equivalent thereof may be used. In general, the fragment that can be used in the assays may have one or more biological activity of FLT3.

In addition, a fusion protein including the fragment or analogue may be used for the screening of the test agent. The functional equivalent of FLT3 is one which includes amino acid deletion, insertion or substitution but retains the same biological activity of FLT3 and, therefore, may be used in the screening method of the present disclosure.

A variety of assays commonly employed in the art may be used to screen the agent regulating FLT3. Specifically, the agent may be screened using a cell-based assay system. For example, in a typical cell-based assay (i.e., second assay) for screening, the activity (e.g., enzymatic activity) of a reporter gene may be measured in the presence of a test agent, and it may be compared with the activity of the reporter gene in the absence of the test agent.

The reporter gene can encode any detectable polypeptide (response or reporter polypeptide) known in the art, e.g., a polypeptide detectable by fluorescence or phosphorescence or by virtue of its enzymatic activity. The detectable response polypeptide may be luciferase, α-glucuronidase, α-galactosidase, chloramphenicol acetyltransferase, green fluorescent protein, enhanced green fluorescent protein, and human alkaline phosphatase, although not being limited thereto.

In the cell-based assay, the test agent (e.g., a peptide or a polypeptide) may also be expressed from a different vector that is also present in a host cell. In some methods, a library of test agents may be encoded by a library of vectors (e.g., a cDNA library). Such a library may be generated using methods well known in the art (see, e.g., Sambrook et al. and Ausubel et al., supra) or may be obtained from a variety of commercial sources.

In addition to the cell-based assay described above, non-cell-based methods can also be used for the screening. These methods include, e.g., mobility shift DNA-binding assay, methylation and uracil interference assay, DNase and hydroxy radical footprinting analysis, fluorescence polarization, and UV crosslinking or chemical cross-linkers. For a general overview, see, e.g., Ausubel et al., supra (chapter 12, DNA-Protein Interactions).

Techniques for isolating co-associating proteins, including nucleic acid- and DNA/RNA-binding proteins, include use of UV crosslinking or chemical cross-linkers, including e.g., cleavable cross-linkers dithiobis(succinimidyl propionate) and 3,3'-dithiobis(sulfosuccinimidyl propionate) (McLaughlin, Am. J. Hum. Genet., 1996; Tang, 1996; Lingner, 1996; Chodosh, 1986).

In another aspect, the present disclosure also provides a composition for predicting a responsiveness of a composition for preventing or treating chronic myelogenous leukemia (CML), which contains an agent which measures the expression level of the FLT3 protein or a gene encoding the same as an active ingredient.

In the present disclosure, the term "the activity of a composition for preventing or treating" means the activity of inhibiting the onset of CML in a normal person who is not definitively diagnosed with CML when administered with a prophylactically effective amount (prophylactic activity), or the activity of inhibiting, alleviating or removing the development of the disease or symptom in vivo when administered to a CML patient with a therapeutically effective amount (therapeutic activity).

In the present disclosure, the term "composition for predicting a responsiveness" means a mixture or a device including a means for measuring the expression level of the FLT3 protein or its gene for predicting the responsiveness of the composition for preventing or treating CML in a subject, and may also be expressed as a "kit for predicting a responsiveness".

In a specific exemplary embodiment of the present disclosure, the agent which measures the expression level of the FLT3 protein may be an antibody or an aptamer binding specifically to the FLT3 protein.

According to the present disclosure, the FLT3 protein may be detected by immunoassay using antigen-antibody reactions for use in analysis of drug reactivity in a subject. The immunoassay may be performed according to various immunoassay or immunostaining protocols developed thus far.

For example, when the method of the present disclosure is carried out by ardioimmunoassay, an antibody labeled with a radioisotopee (e.g., C¹⁴, I¹²⁵, P³² and S³⁵) may be used. In the present disclosure, the antibody specifically recognizing the FLT3 protein is a polyclonal or monoclonal antibody, specifically a monoclonal antibody.

The antibody of the present disclosure may be prepared by methods commonly employed in the art, for example, a hybridoma method (Kohler and Milstein, European Journal of Immunology, 6:511-519 (1976)), a recombinant DNA method (US Patent No. 4,816,567) or a phage antibody library technique (Clackson et al, Nature, 352:624-628 (1991) and Marks et al, J. Mol. Biol., 222:58, 1-597 (1991)). General processes of preparing the antibody are described in detail in Harlow, E. and Lane, D., Using Antibodies: A Laboratory Manual, Cold Spring Harbor Press, New York, 1999; and Zola, H., Monoclonal Antibodies: A Manual of Techniques, CRC Press, Inc., Boca Raton, Florida, 1984.

The drug reactivity can be predicted by analyzing the intensity of the final signal during the immunoassay described above. In other words, if the signal of the FLT3 protein in the subject's sample is stronger than that of the normal sample, it is determined that the drug is less reactive (or resistant).

In the present disclosure, an aptamer binding specifically to the FLT3 protein can also be used instead of the antibody. In the present disclosure, the term "aptamer" refers to a single-stranded nucleic acid (RNA or DNA) molecule or a peptide molecule that binds to a particular target material with high affinity and specificity. The general contents about the aptamer are described in detail in Hoppe-Seyler F, Butz K "Peptide aptamers: powerful new tools for molecular medicine". J Mol Med. 78 (8): 426-30 (2000); and Cohen BA, Colas P, Brent R. "An artificial cell-cycle inhibitor isolated from a combinatorial library". Proc Natl Acad Sci USA. 95 (24): 14272-7 (1998).

In a specific exemplary embodiment of the present disclosure, the agent which measures the expression level of the gene encoding the FLT3 protein is a primer or a probe binding specifically to a nucleic acid molecule of the gene.

In the present disclosure, the term "nucleic acid molecule" is meant to encompass DNA (gDNA and cDNA) and RNA molecules inclusively, and the nucleotides, which are the basic structural units in nucleic acid molecules, may be not only natural nucleotides but also analogues having modified sugar or base residues (Scheit, Nucleotide Analogs, John Wiley, New York (1980); Uhlman and Peyman, Chemical Reviews, 90: 543-584 (1990)).

In the present disclosure, the term "primer" refers to an oligonucleotide which serves as a starting point for synthesis under a condition in which the synthesis of a primer extension product complementary to a nucleic acid chain (template) is induced, i.e., the presence of nucleotides and DNA polymerase, and suitable temperature and pH. Specifically, the primer is a deoxyribonucleotide single chain. The primer used in the present disclosure may include naturally occurring dNMPs (i.e., dAMP, dGMP, dCMP and dTMP), modified nucleotides or non-natural nucleotides. In addition, the primer may also include ribonucleotides.

The primer of the present disclosure may be an extension primer which is annealed to a target nucleic acid and forms a sequence complementary to the target nucleic acid by a template-dependent nucleic acid polymerase, which is extended to the location where an immobilized probe is annealed.

The extension primer used in the present disclosure includes a hybridizable nucleotide sequence complementary to a specific base sequence of a target nucleic acid, e.g., an FLT3-encoding gene. The term "complementary" means that a primer or a probe is complementary enough to selectively hybridize with a target nucleic acid sequence under a certain annealing or hybridization condition. The term "substantially complementary" includes "perfectly complementary" and "completely complementary". In the present disclosure, the term "substantially complementary sequence" includes not only a perfectly matching sequence but also a sequence partially mismatching with a target sequence, within a range where the primer can be annealed to the sequence.

The primer should be long enough so as to prime the synthesis of an extension product in the presence of a polymerase. The suitable length of the primer is determined by a number of factors, e.g., temperature, pH and the source of the primer, but is typically 15-30 nucleotides. Short primer molecules generally require lower temperatures to form a sufficiently stable hybrid complex with a template. The design of such a primer can be easily carried out by those skilled in the art with reference to the target nucleotide sequence, for example, using a program for primer design (e.g., PRIMER 3).

In the present disclosure, the term "probe" refers to naturally occurring or modified monomer or a linear oligomer having linkages, which can be hybridized to a specific nucleotide sequence, including a deoxyribonucleotide and a ribonucleotide. Specifically, the probe is single-stranded for maximum efficiency in hybridization. More specifically, the probe is a deoxyribonucleotide. As the probe of the present disclosure, although a sequence which is perfectly complementary to a specific base sequence of the FLT3-encoding gene may be used, a sequence which is substantially complementary may also be used as long as specific hybridization is not interrupted. Because the stability of a duplex formed by hybridization is determined by the match of sequences at terminals in general, it is preferred to use a probe which is complementary to the 3'-end or 5'-end of the target sequence.

Suitable conditions for hybridization may be determined by referring to Joseph Sambrook, et al., Molecular Cloning, A Laboratory Manual, Cold Spring Harbor Laboratory Press, N.Y. (2001) and Haymes, B. D., et al., Nucleic Acid Hybridization, A Practical Approach, IRL Press, Washington, D.C. (1985).

In another aspect, the present disclosure also provides a composition for diagnosing blast crisis chronic myelogenous leukemia (bc-CML), which contains an agent which measures the expression level of the FLT3 protein or a gene encoding the same as an active ingredient.

Description about the agent which measures the expression level of the FLT3 protein or a gene encoding the same will be omitted to avoid redundancy since it was described in detail above.

In the present disclosure, the term "diagnosis" includes determining the susceptibility of a subject for a specific disease, determining whether a subject has a specific disease, and determining the prognosis of a subject having a specific disease. The present inventors have newly discovered that FLT3, which has been reported to be expressed only in AML, is increased also in CML with the progression of the disease, and is remarkably overexpressed when the disease reaches the blast crisis, and, therefore, its expression level can function as a highly reliable diagnostic marker for bc-CML. Accordingly, the term "diagnosis of blast crisis chronic myelogenous leukemia (bc-CML)" can also be expressed as "diagnosis of the severity of chronic myelogenous leukemia (blast crisis CML; bc-CML)".

In the present disclosure, the term "high expression" or "increase in expression" with regard to the "composition for predicting a responsiveness" or the "composition for diagnosis" means significantly higher expression level of FLT3 as compared to a control group with no tolerance to a CML drug or a control group with no bc-CML. Specifically, the expression level may be increased by about 10% or more, about 20% or more, about 30% or more, about 40% or more, about 50% or more or about 60% or more as compared to the control group, although not being limited thereto.

In another aspect, the present disclosure also provides a composition for inhibiting tolerance to a composition for preventing or treating chronic myelogenous leukemia (CML), comprising an inhibitor against one or more protein selected from the group consisting of JAK (Janus kinase), STAT3 (signal transducer and activator of transcription 3), TAZ (transcriptional coactivator with PDZ-binding motif), TEAD (transcriptional enhancer factor domain) and CD36.

In another aspect, the present disclosure also provides a pharmaceutical composition for preventing or treating chronic myelogenous leukemia, comprising an inhibitor against one or more protein selected from the group consisting of JAK (Janus kinase), STAT3 (signal transducer and activator of transcription 3), TAZ (transcriptional coactivator with PDZ-binding motif), TEAD (transcriptional enhancer factor domain) and CD36 as an active ingredient.

The present inventors have observed that the drug tolerance of chronic myelogenous leukemia, specifically blast crisis chronic myelogenous leukemia, is improved and the progress of the disease is slowed down remarkably by inhibiting the expression of FLT3. Therefore, they have investigated whether a similar therapeutic effect can be achieved by inhibiting the subfactors of FLT3. As a result, they have identified that apoptotic effect is enhanced remarkably and drug tolerance is inhibited when JAK, STAT3, TAZ, TEAD and CD36 are inhibited using AZD1480, C188-9, verteporfin, flufenamic acid and sulfo-N-succinimidyl oleate, respectively, as the FLT3 inhibitor. Accordingly, these inhibitors can be used as an effective composition for preventing or treating chronic myelogenous leukemia or a composition for inhibiting tolerance to the composition, together with the FLT3 inhibitor.

In a specific exemplary embodiment of the present disclosure, the compositions containing the inhibitors are administered together with a tyrosine kinase inhibitor. The co-administration may be made simultaneously or sequentially with appropriate order and time interval as either a combined composition of the two active ingredients or as individual pharmaceutical compositions.

In another aspect, the present disclosure also provides a method for inhibiting tolerance to a composition for preventing or treating chronic myelogenous leukemia (CML), comprising administering to a subject in need thereof a composition comprising an FLT3 (FMS-like tyrosine kinase 3) inhibitor as an active ingredient.

In another aspect, the present disclosure also provides a method for preventing or treating chronic myelogenous leukemia, comprising administering to a subject in need thereof a composition comprising an FLT3 (FMS-like tyrosine kinase 3) inhibitor as an active ingredient to a subject.

In another aspect, the present disclosure also provides a method for predicting a responsiveness for a composition for preventing or treating chronic myelogenous leukemia (CML) in a subject, comprising measuring the expression level of the FLT3 protein or a gene encoding the same in the subject.

In another aspect, the present disclosure also provides a method for diagnosing blast crisis chronic myelogenous leukemia (bc-CML), comprising measuring the expression level of the FLT3 protein or a gene encoding the same in a subject.

In another aspect, the present disclosure also provides a method for inhibiting tolerance to a composition for preventing or treating chronic myelogenous leukemia (CML), comprising administering a composition containing an inhibitor against one or more protein selected from the group consisting of JAK (Janus kinase), STAT3 (signal transducer and activator of transcription 3), TAZ (transcriptional coactivator with PDZ-binding motif), TEAD (transcriptional enhancer factor domain) and CD36 as an active ingredient to a subject in need thereof.

In another aspect, the present disclosure also provides a method for preventing or treating chronic myelogenous leukemia, comprising administering a composition containing an inhibitor against one or more protein selected from the group consisting of JAK (Janus kinase), STAT3 (signal transducer and activator of transcription 3), TAZ (transcriptional coactivator with PDZ-binding motif), TEAD (transcriptional enhancer factor domain) and CD36 as an active ingredient to a subject in need thereof.

### [Mode for Invention]

Hereinafter, the present disclosure is described in more detail through examples. However, it is obvious that the present disclosure is not limited by the examples.

### Test Example 1: Measurement of mRNA and protein expression

The expression pattern of FLT3 and TAZ mRNAs in patients with chronic myelogenous leukemia depending on phases were analyzed based on the previously reported research results (Radich JP et al. Proc Natl Acad Sci USA. 21; 103 (8): 2794-2499 (2006)).

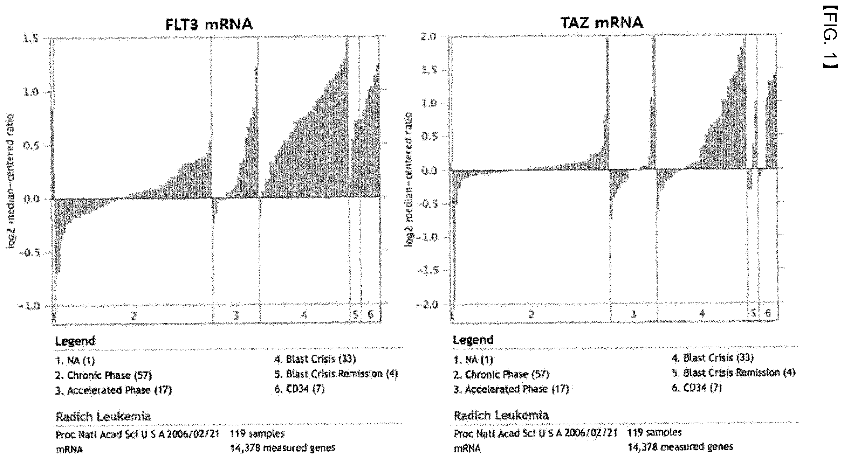

Referring to FIG. 1, the expression level of FLT3 and TAZ mRNAs was increased rapidly as chronic myelogenous leukemia progressed from the chronic phase to the accelerated phase and to the blast crisis.

This result suggests the close relationship between drug tolerance, which increases rapidly with the progression of CML, and FLT3.

### Test Example 2: Drug tolerance test

Drug tolerance was tested using FLT3-transformed K562 cells (ATCC, CCL-243) mimicking the blast crisis of chronic myelogenous leukemia. Drugs and antibodies were purchased from the following suppliers: imatinib (Selleckchem, Cat. No. S2475), nilotinib (Selleckchem, Cat. No. S1033), dasatinib (Santa Cruz, CAS 302962-49-8), anti-TAZ antibody (Santa Cruz, Cat. No. sc-101199), anti-FLT3 antibody (Cell signaling, Cat. No. 3462), anti-GAPDH antibody (Santa Cruz, Cat. No. sc-32233).

After administering three anticancer drugs against chronic myelogenous leukemia for 17 days to the FLT3-transformed K562 cells, the surviving cells were counted (Table 1).

**[Table 1]**

| | | Cell number (x 10⁵) | | | | | | |
|---|---|---|---|---|---|---|---|---|
| Treatment | K562 | Day 0 | Day 5 | Day 9 | Day 11 | Day 13 | Day 15 | Day 17 |
| 1 µM STI | WT | 1 | 0.485 | 0.058 | 0.52 | 0.175 | 0 | 0 |
| | FLT3 | 1 | 1.013 | 1.905 | 1.82 | 2.14 | 3.17 | 14.3 |
| 1 µM dasatinib | WT | 1 | 0.8075 | 0.35 | 0.47 | 0 | 0 | 0 |
| | FLT3 | 1 | 1.57 | 2.87 | 2.23 | 2.26 | 1.76 | 9.33 |
| 1 µM nilotinib | WT | 1 | 0.9675 | 0.23 | 0.76 | 0.09 | 0 | 0 |
| | FLT3 | 1 | 2.0975 | 1.38 | 2.64 | 2.84 | 4.52 | 17.1 |

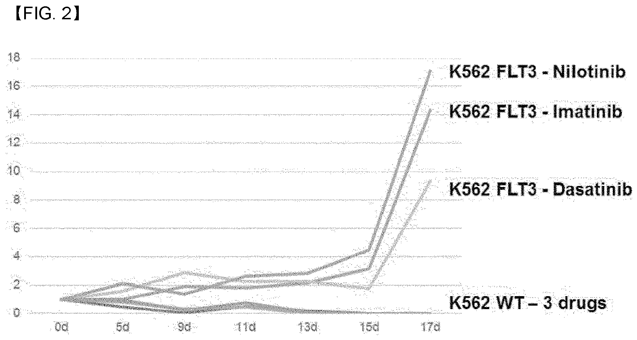

Referring to FIG. 2, all the wild-type (WT) K562 cells treated with the anticancer drugs were killed due to no resistance to the drugs. In contrast, a large number of the FLT3-transformed K562 cells survived after treatment with the anticancer drugs due to strong drug resistance.

In addition, the expression level of TAZ and FLT3 was measured through western blot.

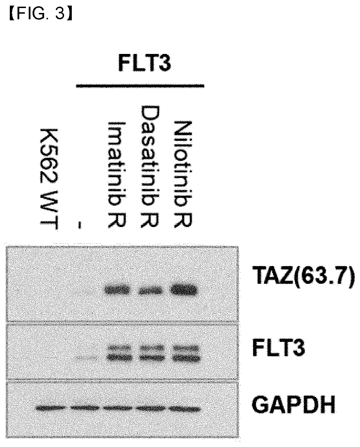

Referring to FIG. 3, whereas TAZ and FLT3 were not expressed in the wild-type (WT) K562 cells, the expression of FLT3 and TAZ was remarkably increased in the FLT3-transformed K562 cells and the expression of FLT3 and TAZ was increased further when drug resistance was acquired.

This result suggests that FLT3 is an important factor inducing drug tolerance in chronic myelogenous leukemia and that the expression of FLT3 and TAZ is closely related with drug tolerance.

That is to say, it can be seen that the cells acquire drug resistance since FLT3 induces and increases the expression of TAZ.

### Test Example 3: Test of drug tolerance-inhibiting activity

It was investigated whether FLT3 inhibitors used as therapeutic agents for AML have anticancer activity against chronic myelogenous leukemia (CML).

The effect of FLT3 inhibitors on FLT3-transformed K562 cells having resistance to three drugs (imatinib, dasatinib and nilotinib) was investigated.

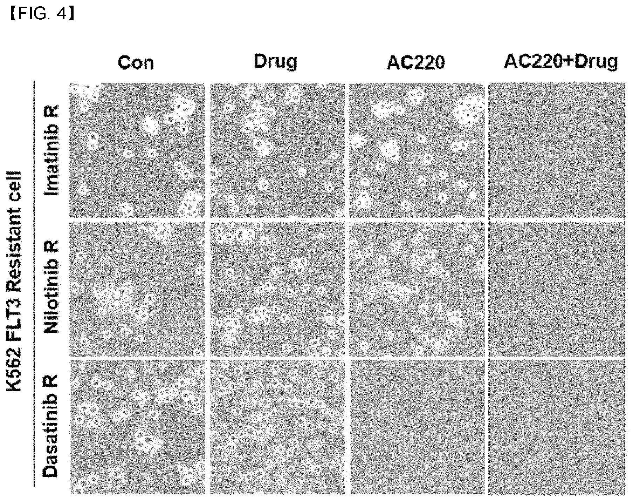

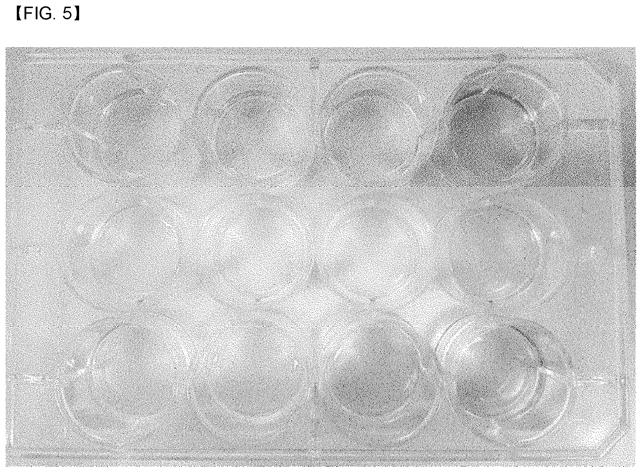

Referring to FIGS. 4 and 5, the cells treated only with the FLT3 inhibitor, quizartinib (AC220), or only with the therapeutic agents for CML (imatinib, dasatinib and nilotinib) were hardly killed, whereas the resistant cells were killed completely when they were treated with the FLT3 inhibitor, quizartinib, and the therapeutic agents for CML together.

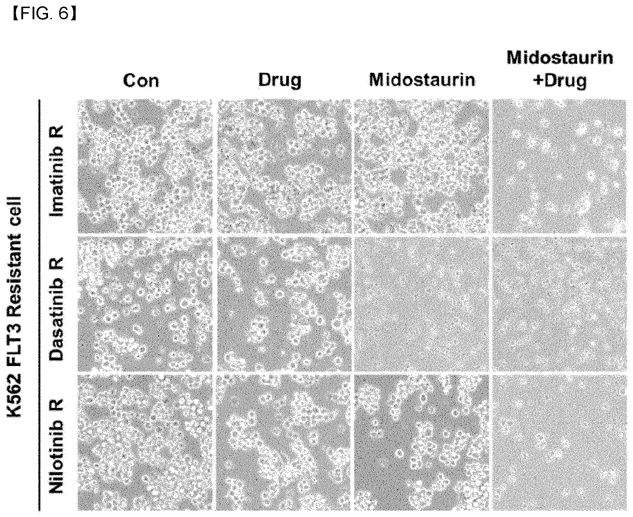

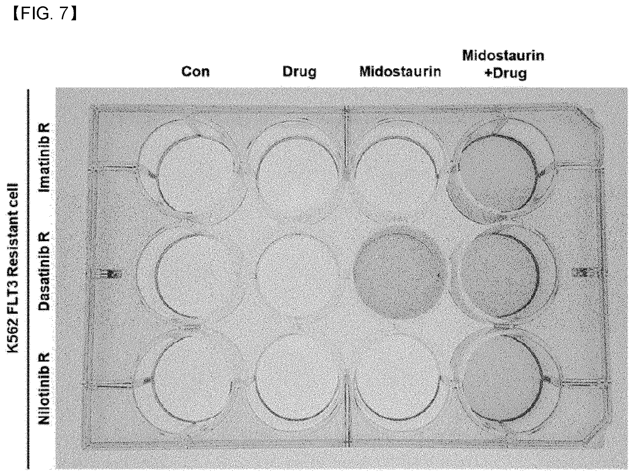

Referring to FIGS. 6 and 7, the cells treated only with the FLT3 inhibitor, midostaurin, or only with the therapeutic agents for CML (imatinib, dasatinib and nilotinib) were hardly killed, whereas the resistant cells were killed effectively when they were treated with the FLT3 inhibitor, midostaurin, and the therapeutic agents for CML together.

In particular, the expression of the TAZ transcription factor was inhibited when the cells were treated with the FLT3 inhibitor and the therapeutic agents for CML together, suggesting that drug tolerance was removed.

This result suggests that, although the FLT3 inhibitor does not exhibit a therapeutic effect for chronic myelogenous leukemia directly, it can alleviate or remove the resistance of cells to drugs for CML.

### Test Example 4: Test of drug tolerance-inhibiting activity of FLT3 inhibitor

In order to test whether FLT3 inhibitors other than quizartinib also have comparable effect of inhibiting drug tolerance, the same experiment was conducted using anti-FLT3 antibody, siRNA, dovitinib, amuvatinib, tandutinib, gilteritinib and pacritinib.

As a result, the inhibitors showed similar or comparable drug tolerance.

This result suggests that FLT3 is directly related with the drug tolerance of CML and that the drug tolerance of CML can be interrupted by inhibiting the activity or expression of FLT3.

### Test Example 5: Test of drug tolerance and apoptotic activity of FLT3 inhibitor

In order to investigate whether FLT3 inhibitors including quizartinib have comparable or similar drug tolerance-inhibiting and apoptotic effects, the effect of co-administration of ponatinib (Selleckchem, Cat. No. S1490), quizartinib (Selleckchem, Cat. No. S1526), midostaurin (Cayman, Cat. No. 20685-11-2), sorafenib (Cayman, Cat. No. 284461-73-0), gilteritinib (Cayman, Cat. No. 1254053-43-4) and crenolanib (Cayman, Cat. No. 670220-88-9) with imatinib was investigated by MTT assay.

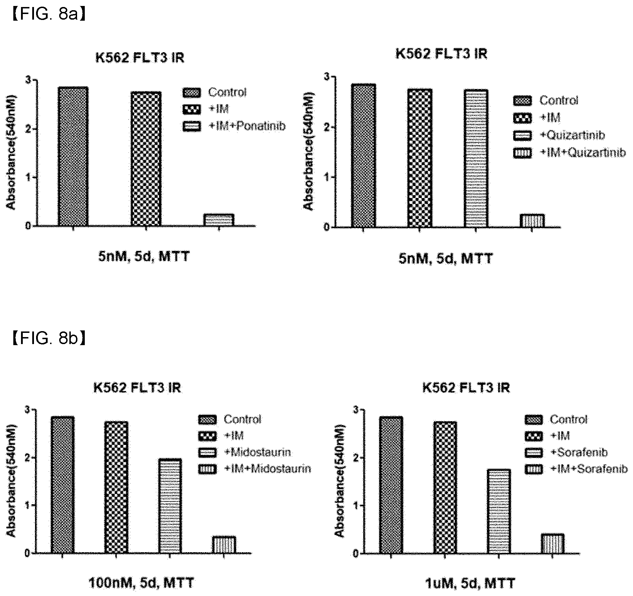

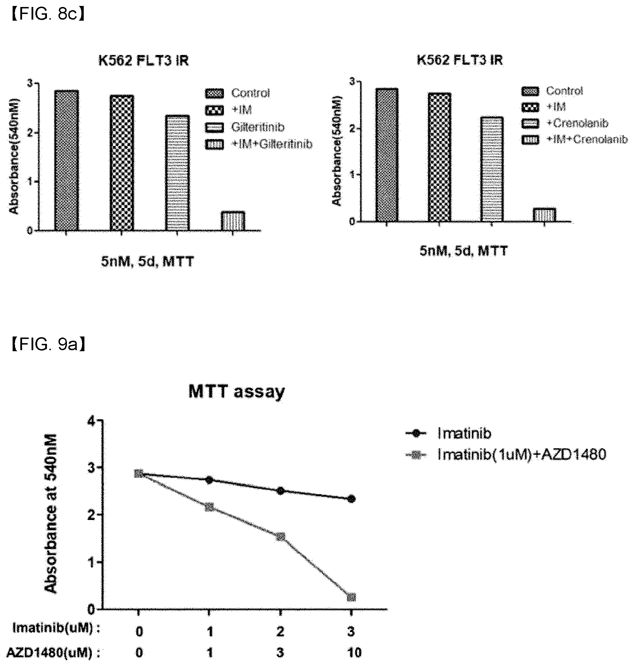

The FLT3-transformed K562 cells having resistance to imatinib (Selleckchem, Cat. No. S2475), established in Test Example 3, were seeded onto a 12-well cell culture plate (Falcon, Cat. No. 353043), with a density of 5x10⁴ cells/mL per well. The cells were cultured using DMEM (Hyclone) containing 10% FBS (Gibco). The cells were divided into a control group not treated with a drug, a group treated with 1 µM imatinib, and a group co-treated with 1 µM imatinib and an FLT3 inhibitor. The concentration of the FLT3 inhibitor was as follows. Ponatinib, quizartinib, gilteritinib and crenolanib: 5 nM; midostaurin: 100 nM; sorafenib: 1 µM. 5 days later, after adding 100 µL of an MTT labeling reagent included in a cell proliferation kit (Roche) to each well for 2 hours and then treating with 1 mL of a solubilization solution for 24 hours, absorbance was measured at a wavelength of 540 nM using a multiplate reader (FIGS. 8a-8c). As seen from FIGS. 8a-8c, all the FLT3 inhibitors showed remarkably improved apoptotic effect as in Test Example 3 when co-treated with imatinib, whereas the FLT3-overexpressed K562 cells could not be killed only with imatinib.

### Test Example 6: Test of drug tolerance inhibition and apoptotic activity of inhibitors against FLT3 subfactors

The FLT3-JAK-STAT3-TAZ-TEAD-CD36 signal transduction of chronic myelogenous leukemia elucidated by the experiment using quizartinib increases drug tolerance. In order to investigate whether the inhibition of JAK-STAT3-TAZ-TEAD-CD36, which are subfactors of FLT3, in the signal transduction system exhibits drug tolerance-inhibiting and apoptotic effects as in Test Example 3, MTT assay was conducted using the following inhibitors.

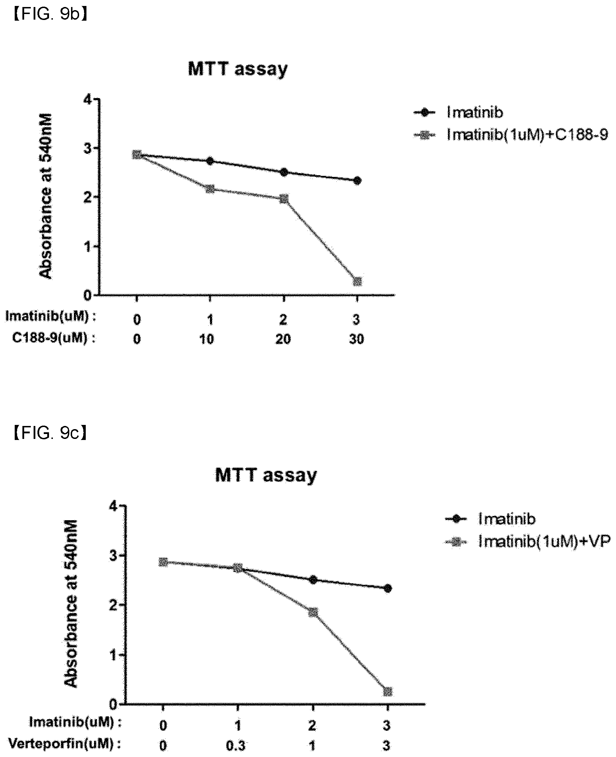

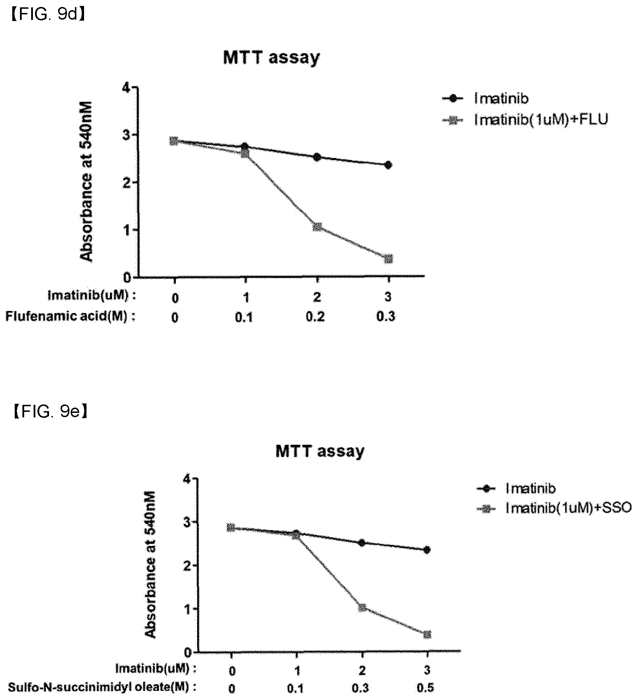

AZD1480 (Selleckem, Cat. No. S2162) was used as a JAK inhibitor, C188-9 (Selleckem, Cat. No. S8605) was used as a STAT3 inhibitor, verteporfin (Selleckem, Cat. No. S1786) was used as a TAZ inhibitor, flufenamic acid (Selleckem, Cat. No. S4268) was used as a TEAD inhibitor, and sulfo-N-succinimidyl oleate (Cayman, Cat. No. 11211) was used as a CD36 inhibitor. After seeding the cells established in Test Example 3 onto a plate in the same manner as in Test Example 5, the cells were divided into a control group not treated with an inhibitor, a group treated with imatinib (1, 2, 3 µM), a group treated with imatinib (1 µM) and AZD1480 (1, 3, 10 µM), a group treated with imatinib (1 µM) and C188-9 (0.3, 1, 3 µM), a group treated with imatinib (1 µM) and verteporfin (0.3, 1, 3 µM), a group treated with imatinib (1 µM) and flufenamic acid (0.1, 0.2, 0.3 M), and a group treated with imatinib (1 µM) and sulfo-N-succinimidyl oleate (0.1, 0.3, 0.5 M). After treating with each inhibitor for 4 days, MTT assay was conducted in the same manner as in Test Example 5 and absorbance was measured at 540 nm using a multiplate reader (FIG. 9).

As seen from FIGS. 9a-9e, all the inhibitors against the subfactors of FLT3 (AZD1480, C188-9, verteporfin, flufenamic acid and sulfo-N-succinimidyl oleate) remarkably enhanced apoptotic effect and inhibited drug tolerance when co-treated with imatinib, like the FLT3 inhibitor.

### Test Example 7: Test of drug tolerance inhibition and apoptotic activity of treatment with inhibitor against FLT3 subfactor alone

It was confirmed through Test Example 6 that the co-treatment of the inhibitor against JAK, STAT3, TAZ, TEAD or CD36 with imatinib exhibits drug tolerance-inhibiting and apoptotic effects. In order to investigate whether the inhibitors also exert significant effect of preventing and treating chronic myelogenous leukemia when treated alone, MTT assay was conducted after treating cells with the inhibitor alone.

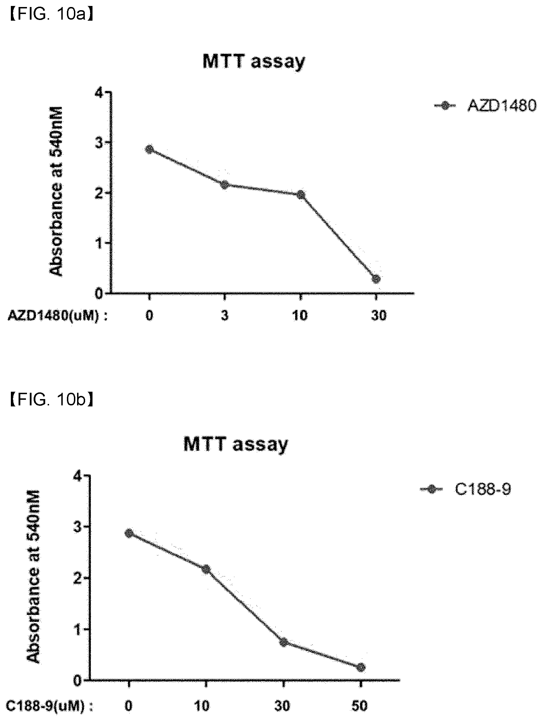

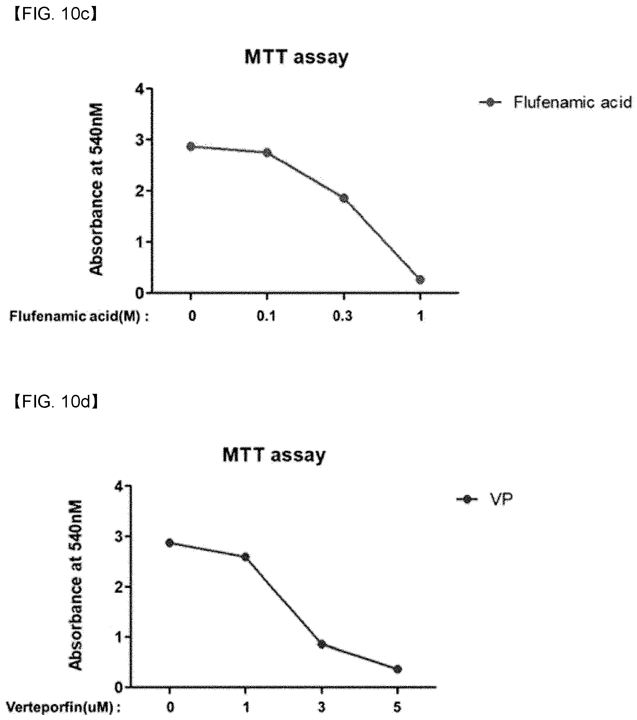

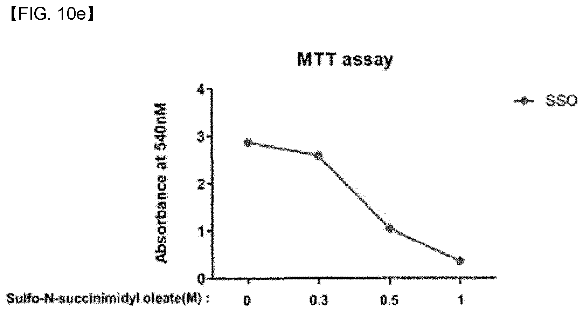

The same inhibitors used in Test Example 6 were used. After seeding cells onto a 12-well plate under the same condition as in Test Example 5, the cells were treated with each inhibitor at different concentrations for 3 days without imatinib. The concentrations of the drugs used are as follows: AZD1480 (3, 10, 30 µM), C188-9 (10, 30, 50 µM), verteporfin (1, 3, 5 µM), flufenamic acid (0.1, 0.3, 1 µM), sulfo-N-succinimidyl oleate (0.3, 0.5, 1 M). 3 days after the single treatment, absorbance was measured at a wavelength of 540 nM using the cell proliferation kit and the multiplate reader used in Test Example 5 (FIGS. 10a-10e). As seen from FIGS. 10a-10e, all the inhibitors against the subfactors of FLT3 remarkably enhanced apoptotic effect and inhibited drug tolerance of the FLT3-overexpressed cells when treated alone. Accordingly, the inhibitors can be used, not only as therapeutic adjuvants or drug tolerance inhibitors, but also as compositions having pharmacological effect for preventing or treating CML by themselves.

Those having ordinary knowledge in the art to which the present disclosure belongs will understand that the foregoing description of the present disclosure is for illustration only and it can be easily modified into other forms without changing the technical idea or essential features of the present disclosure. Therefore, it should be understood that the exemplary embodiments described above are illustrative, not limitative.

The scope of the present disclosure is defined by the appended claims, and it should be understood that the meaning and scope of the claims and all variations or modified forms derived from the equivalent concept thereof are encompassed within the scope of the present disclosure.

## Claims

1. A composition for inhibiting tolerance to a composition for preventing or treating chronic myelogenous leukemia (CML), comprising an FLT3 (FMS-like tyrosine kinase 3) inhibitor as an active ingredient.

2. The composition according to claim 1, wherein the FLT3 inhibitor is one or more inhibitor selected from the group consisting of ponatinib, quizartinib, midostaurin, dovitinib, amuvatinib, tandutinib, sorafenib, gilteritinib, crenolanib and pacritinib.

3. The composition according to claim 1, wherein the chronic myelogenous leukemia (CML) is blast crisis chronic myelogenous leukemia (bc-CML).

4. The composition according to claim 1, wherein the composition for preventing or treating chronic myelogenous leukemia (CML) is a tyrosine kinase inhibitor.

5. The composition according to claim 4, wherein the tyrosine kinase inhibitor is one or more inhibitor selected from the group consisting of imatinib, dasatinib, nilotinib, bosutinib and ponatinib.

6. A pharmaceutical composition for preventing or treating chronic myelogenous leukemia, comprising an FLT3 inhibitor as an active ingredient.

7. The composition according to claim 6, wherein the FLT3 inhibitor is one or more inhibitor selected from the group consisting of ponatinib, quizartinib, midostaurin, dovitinib, amuvatinib, tandutinib, sorafenib, gilteritinib, crenolanib and pacritinib.

8. The composition according to claim 6, wherein the composition is administered together with a tyrosine kinase inhibitor.

9. The composition according to claim 8, wherein the tyrosine kinase inhibitor is one or more inhibitor selected from the group consisting of imatinib, dasatinib, nilotinib, bosutinib and ponatinib.

10. The composition according to claim 6, wherein the chronic myelogenous leukemia (CML) is drug-tolerant.

11. A method for screening a composition for inhibiting tolerance to a composition for preventing or treating chronic myelogenous leukemia (CML), comprising:
(a) contacting a biological sample comprising FLT3-expressing cells with a test agent; and
(b) measuring the expression level or activity of the FLT3 in the sample,
wherein, if the expression level or activity of the FLT3 is decreased, the test agent is determined as a composition for inhibiting tolerance to a composition for preventing or treating chronic myelogenous leukemia.

12. A composition for predicting a responsiveness of a composition for preventing or treating chronic myelogenous leukemia (CML), comprising an agent which measures the expression level of FLT3 protein or a gene encoding the same as an active ingredient.

13. The composition according to claim 12, wherein the agent which measures the expression level of the FLT3 protein is an antibody or an aptamer binding specifically to the FLT3 protein.

14. The composition according to claim 12, wherein the agent which measures the expression level of the gene encoding the FLT3 protein is a primer or a probe binding specifically to a nucleic acid molecule of the gene.

15. A composition for diagnosing blast crisis chronic myelogenous leukemia (bc-CML), comprising an agent which measures the expression level of FLT3 protein or a gene encoding the same as an active ingredient.

16. A composition for inhibiting tolerance to a composition for preventing or treating chronic myelogenous leukemia (CML), comprising an inhibitor against one or more protein selected from the group consisting of JAK (Janus kinase), STAT3 (signal transducer and activator of transcription 3), TAZ (transcriptional coactivator with PDZ-binding motif), TEAD (transcriptional enhancer factor domain) and CD36 as an active ingredient.

17. A pharmaceutical composition for preventing or treating chronic myelogenous leukemia, comprising an inhibitor against one or more protein selected from the group consisting of JAK (Janus kinase), STAT3 (signal transducer and activator of transcription 3), TAZ (transcriptional coactivator with PDZ-binding motif), TEAD (transcriptional enhancer factor domain) and CD36 as an active ingredient.

18. The composition according to claim 17, wherein the composition is administered together with a tyrosine kinase inhibitor.
